Europäisches Patentamt

European Patent Office

Office européen des brevets

(11) Veröffentlichungsnummer: **0 275 956**

**A2**

## EUROPÄISCHE PATENTANMELDUNG

(21) Anmeldenummer: **88100612.6**

(22) Anmeldetag: **18.01.88**

(51) Int. Cl.⁴: **A61B 5/10**

(30) Priorität: **22.01.87 DE 3701820**
**20.02.87 DE 3705493**

(43) Veröffentlichungstag der Anmeldung:
**27.07.88 Patentblatt 88/30**

(84) Benannte Vertragsstaaten:
**AT BE CH DE ES FR GB IT LI NL SE**

(71) Anmelder: **Kern, Günter**
**Am Datzmann 63**
**D-8242 Bischofswiesen(DE)**

Anmelder: **Kranz, Rainer**
**Rainweg 3**
**D-8261 Tyrlaching(DE)**

(72) Erfinder: **Kern, Günter**
**Am Datzmann 63**
**D-8242 Bischofswiesen(DE)**
Erfinder: **Kranz, Rainer**
**Rainweg 3**
**D-8261 Tyrlaching(DE)**

(74) Vertreter: **Gossel, Hans K., Dipl.-Ing. et al**
**Rechtsanwälte E. Lorenz - B. Seidler M.**
**Seidler - Dipl.-Ing. H.K. Gossel Dr. I. Philipps**
**- Dr. P.B. Schäuble Dr. S. Jackermeier -**
**Dipl.-Ing. A. Zinnecker**
**Widenmayerstrasse 23 D-8000 München**
**22(DE)**

(54) **Medizinisches Diagnosegerät.**

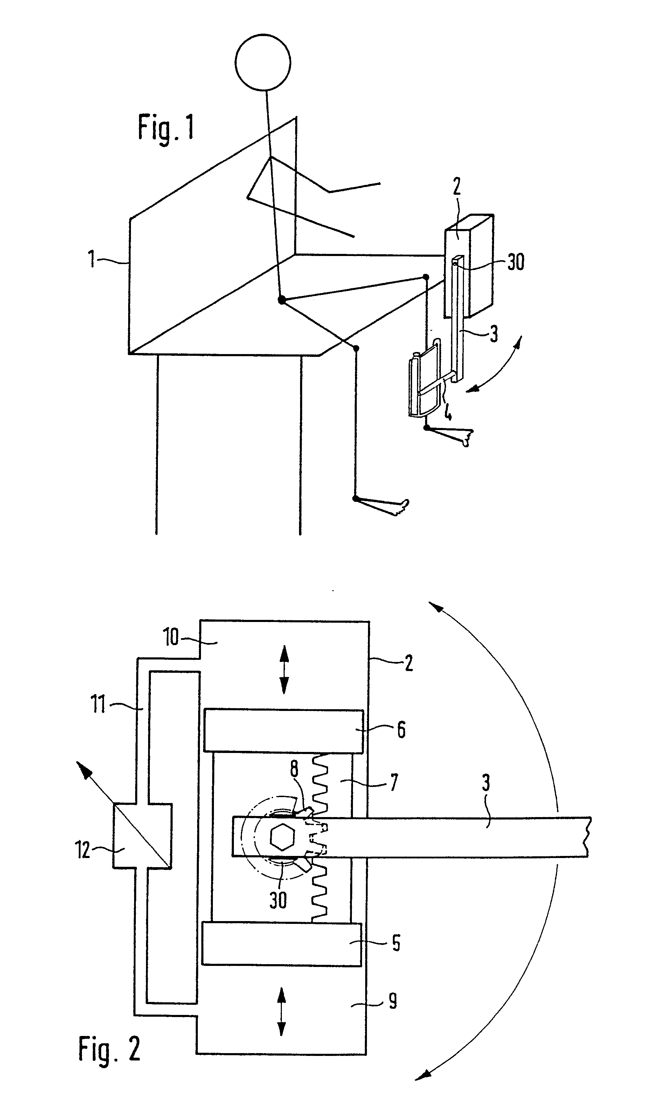

(57) Ein medizinisches Diagnosegerät besitzt einen an einer Schwenkachse (30) befestigten Hebel (3), der zur Erzeugung eines Reaktionsmoments mit den Kolben (5, 6) eines Hydraulikzylinders (2) so verbunden ist, daß eine alternierende Drehbewegung des Hebels (3) in eine translatorische hin-und hergehende Bewegung des Kolbens (5, 6) umgewandelt wird. Eine Meßeinrichtung dient zum Messen des in dem Hydraulikzylinder (2) herrschenden Drucks. Eine Anzeigeeinrichtung dient zum Anzeigen des gemessenen Drucks. Damit ein derartiges medizinisches Diagnosegerät einfach und raumsparend aus-gebildet werden kann, durchsetzt die Schwenkachse (30) den Zylinder (2) quer und ist die Schwenkachse (30) im Innern des Zylinders (2) mit den Kolben (5, 6) durch ein Getriebe (7, 8) verbunden (Fig. 2).

Fig. 2

### Medizinisches Diagnosegerät

Die Erfindung betrifft ein medizinisches Diagnosegerät mit einem an einer Schwenkachse befestigten Hebel, der zur Erzeugung eines Reaktionsmoments mit dem Kolben eines Hydraulikzylinders so verbunden ist, daß eine alternierende Drehbewegung des Hebels in eine translatorische hin-und hergehende Bewegung des Kolbens umgewandelt wird, mit einer Meßeinrichtung zum Messen des in dem Hydraulikzylinder herrschenden Drucks und mit einer Anzeigeeinrichtung zum Anzeigen des gemessenen Drucks.

Ein derartiges medizinisches Diagnosegerät ist aus der PCT-Veröffentlichung WO 86/06947 bekannt. Der Kolben treibt dabei eine Kette an, die über Zahnräder oder Kettenräder läuft, von denen eines mit dem Hebel gekoppelt ist. Der Aufbau des vorbekannten medizinischen Diagnosegeräts ist daher kompliziert und platzaufwendig.

Aufgabe der Erfindung ist es, ein medizinisches Diagnosegerät der eingangs angegebenen Art zu schaffen, das einfach und raum sparend aufgebaut ist.

Erfindungsgemäß wird diese Aufgabe dadurch gelöst, daß die Schwenkachse den Zylinder quer durchsetzt und daß die Schwenkachse im Innern des Zylinders mit dem Kolben durch ein Getriebe verbunden ist. Die das Reaktionsmoment erzeugende und aus dem Hebel und dem Hydraulikzylinder bestehende Einheit weist dadurch eine einfache, stabile und raumsparende Konstruktion auf.

Die Person, deren Gelenkstruktur oder Muskelfaserstruktur diagnostiziert werden soll, betätigt den an der Schwenkachse befestigten Hebel mit einem Körperteil, dessen Gelenkstruktur oder Muskelfaserstruktur untersucht werden soll. Während der Bewegung wird der in dem Hydraulikzylinder herrschende Druck gemessen und von der Anzeigeeinrichtung angezeigt. Aus dem Druckverlauf kann dann die Gelenkstruktur oder die Muskelfaserstruktur diagnostiziert werden.

Der Kolben des Hydraulikzylinders kann ein doppelt wirkender Kolben sein, und es kann ein eine Drosseleinrichtung aufweisender Kanal vorgesehen sein, der die durch den doppelt wirkenden Kolben getrennten und mit Flüssigkeit gefüllten Zylinderkammern miteinander verbindet. Dadurch ist es möglich, sowohl der hingehenden Bewegung als auch der hergehenden Bewegung des Hebels und damit des Kolbens einen Widerstand entgegenzusetzen.

In einer vorteilhaften Weiterbildung der Erfindung ist eine Winkelmeßvorrichtung zur Messung des Drehwinkels der Schwenkachse vorgesehen. Hierdurch kann der Verlauf des Druckes und damit des Drehmoments über den Drehwinkel ermittelt werden, was weitere diagnostische Möglichkeiten eröffnet.

Der mit der Drosseleinrichtung versehene Kanal kann in der Zylinderwandung angeordnet sein, was zu einer weiteren Verein fachung und Raumersparnis führt. Das Getriebe läßt sich in vorteilhafter und einfacher Weise dadurch bilden. daß der Kolben zwischen den Kolbenscheiben mit einer achsparallelen Zahnstange versehen ist, mit der ein mit der Schwenkachse fest verbundenes Ritzel kämmt. Das Getriebe kann auch durch eine Kurbel und eine Pleuelstange oder Exzenter gebildet werden. Zweckmäßigerweise ist der Zylinder mit dem aus einem Stuhl, einer Liege oder dergleichen bestehenden Diagnosegerät derart verbunden, daß die Schwenkachse im wesentlichen mit der Achse des Gelenks oder Beugungszentrums des zu diagnostizierenden Gelenks oder Körperteils fluchtet.

Der Schwenkwinkel des Hebels kann durch Anschläge einstellbar sein, um zu große Bewegungen zu verhindern. Weiterhin ist die Länge des Hebelarms des Hebels zweckmäßigerweise einstellbar.

Um für die hingehende Bewegung einerseits und für die hergehende Bewegung andererseits verschiedene Reaktionsmomente einstellen zu können, ist in den Endbereichen des Kanals je ein einstellbares Drosselventil angeordnet, besteht der Kolben aus zwei starr miteinander verbundenen Kolbenscheiben, die zwischen sich eine Kolbenkammer begrenzen, ist in jeder Kolbenscheibe ein einen Durchfluß in die angrenzende Zylinderkammer gestattendes Rückschlagventil angeordnet und ist der Kanal zwischen den Drosselventilen mit einer durch die Zylinderwandung hindurch in die Kolbenkammer mündenden Leitung versehen.

Eine vorteilhafte Weiterbildung ist dadurch gekennzeichnet, daß der Hebel in einer Richtung senkrecht zu seiner Bewegungsrichtung auslenkbar ist. Den Besonderheiten des menschlichen Bewegungsapparates entsprechend beobachtet man beispielsweise bei der Bewegung des menschlichen Kniegelenks, daß sich der Unterschenkel während des Überganges von der Beugung in die Streckung nicht nur auf einer senkrecht zur Gelenkachse (Dreh achse) stehenden Ebene bewegt, sondern daß er mit zunehmender Streckung auch noch zur Körperinnenseite wandert. Die Ursachen hierfür sind, wie medizinisch bekannt ist, die bei jedem Menschen verschiedenen, individuellen Achsverschiebungen von Ober-zu Unterschenkel. Im medizinischen Sprachgebrauch wird dieser Effekt als "zwangsläufige Innenrotation" des Unterschenkels bei zunehmender Streckung des Kniegelenks bezeichnet.

Um die Belastung des Knorpel-und Bandapparates der zu diagnostizierenden Person vermindern zu können, ist der Hebel in einer Richtung senkrecht zu seiner Bewegungsrichtung auslenkbar. Dadurch kann das erfindungsgemäße Diagnosegerät den natürlichen Bewegungsabläufen des menschlichen Körpers bzw. der Gelenke des menschlichen Körpers folgen. Das erfindungsgemäße Diagnosegerät kann also einseitige Belastungen in den Gelenken der zu diagnostizierenden Person aufnehmen, gleichmäßig verteilen und dadurch den Knorpel-und Bandapparat schonen, also Reiz-bzw. Entzündungserscheinungen am Gelenk verhindern.

Die Auslenkbarkeit des Hebels in einer Richtung senkrecht zu seiner Bewegungsrichtung kann auf verschiedene Weise erreicht werden. Es ist möglich, daß der Hebel an der Schwenkachse um eine senkrecht zur Schwenkachse und senkrecht zum Hebel verlaufende Drehachse schwenkbar gelagert ist. Weiterhin kann die Schwenkachse um eine senkrecht zur Schwenkachse und im wesentlichen parallel zum Hebel verlaufende Drehachse - schwenkbar gelagert sein. Der Hebel kann mit der Schwenkachse drehfest und in Richtung der Schwenkachse längsverschieblich verbunden sein. Mit dem Hebel kann ein parallel zu Schwenkachse verlaufendes Querstück verbunden sein, längs dem eine Beinaufnehmerschale längsverschieblich geführt ist. Die Drehachse kann im Abstand zur Schwenkachse angeordnet sein.

In einer weiteren vorteilhaften Weiterbildung ist eine Verarbei tungseinrichtung zur Verarbeitung der Druckmeßwerte und/oder der Winkelmeßwerte vorgesehen. Durch diese Verarbeitungseinrichtung können gemessene Werte mit bereits früher gemessenen Werten und/oder mit vorgegebenen Meßwerten verglichen werden. Weiterhin ist eine Verarbeitung der Meßwerte zu beliebigen anderen diagnostischen Zwecken möglich.

Die Anzeigeeinrichtung kann aus einem Oszillographen und/oder einem Meßschreiber bestehen.

In weiterer Ausgestaltung der Erfindung ist der Zylinder mit der Schwenkwelle eines zweiten gleichartigen, an dem Diagnosegerät befestigten Zylinder verbunden. Hierbei kann die Schwenkwelle des zweiten Zylinders mit der Längsachse der ersten Zylinders fluchten; diese beiden Achsen können jedoch auch im Winkel zueinander angeordnet sein. Diese Ausgestaltung ermöglicht gleichsam kardanische räumliche Bewegungen, wie sie beispielsweise zur Diagnose von Hüft-oder Schultergelenken, aber auch von anderen Gelenken zweckmäßig sind. Eine derartige räumliche Bewegung wird durch die besonders platzsparende Bauweise des erfindungsgemäßen Diagnosegeräts ermöglicht.

Weitere vorteilhafte Weiterbildungen sind in den Unteransprüchen beschrieben.

Ein Ausführungsbeispiel der Erfindung wird nachstehend anhand der beigefügten Zeichnungen im einzelnen beschrieben. In diesen zeigt:

Fig. 1 das aus einem Stuhl bestehende Diagnosegerät in perspektivischer. schematischer Darstellung,

Fig. 2 eine schematische Darstellung des mit dem Hebel versehenen Hydraulikzylinders,

Fig. 3 bis 5 den Hydraulikzylinder in zwei Seitenansichten und in Draufsicht,

Fig. 6 bis 8 den Kolben in zwei Seitenansichten und in Draufsicht,

Fig. 9 die Schwenkwelle und den Hebel in perspektivischer Darstellung

Fig. 10 den Zylinder, die Schwenkwelle und den Hebel in perspektivischer Darstellung,

Fig. 11 die Schwenkwelle und den Hebel in einer weiteren Darstellung,

Fig. 12 die Schwenkwelle und den Hebel in einer weiteren Darstellung,

Fig. 13 eine weitere Ausführungsform der Erfindung,

Fig. 14 eine weitere Ausführungsform der Erfindung,

Fig. 15 eine weitere Ausführungsform der Erfindung,

Fig. 16 ein Diagramm eines gemessenen Druckverlaufes,

Fig. 17 ein weiteres Diagramm der in Fig. 16 dargestellten Art,

Fig. 18 eine schematische Darstellung verschiedener Winkelstellungen des Hebels,

Fig. 19 eine schematische Darstellung der in Fig. 18 gezeigten Winkelstellungen,

Fig. 20 eine schematische Darstellung des prinzipiellen Aufbaus eines Auswertegerätes,

Fig. 21 eine mit dem Diagnosegerät und dem zugehörigen Auswertegerät hergestellte Original-Messkurve,

Fig. 22 eine mit einem vorbekannten Gerät hergestellte Meßkurve,

Fig. 23 zwei weitere mit dem Diagnosegerät und dem zugehörigen Auswertegerät hergestellte Meßkurven.

Nach dem Ausführungsbeispiel der Fig. 1 besteht das Diagnosegerät aus einem Stuhl 1, der nur durch Sitzfläche, Rückenlehne und Beine angedeutet ist. An der Sitzfläche ist der Hydraulikzylinder 2 mit dem Schwenkhebel 3 in der Weise befestigt, daß die Schwenkachse 30 in etwa mit der durch die beiden Kniegelenke des Patienten gezogenen Linie fluchtet. An dem Schwenkhebel 3, der in nicht dargestellter Weise längenverstellbar ist, ist endseitig ein Querstück 4 angesetzt, das Bandagen zur Befestigung an dem Unterschenkel das Patienten trägt.

Der Stuhl 1 ist weiterhin mit nicht dargestellten Befestigungsgurten für den nur durch Striche dargestellten Patienten versehen.

Um die Schwenkachse 30 des Hydraulikzylinders 2 bezüglich der Kniegelenke des Patienten zu justieren, kann der Hydraulikzylinder 2 an dem Stuhl mittels einer Kreuzführung befestigt sein, die eine Bewegung längs zweier vorzugsweise senkrecht zueinander stehender Linien ermöglicht. Damit ist sowohl eine Bewegungsmöglichkeit nach vorne und hinten als auch eine Bewegungsmöglichkeit nach oben und unten gegeben. Stattdessen kann auch die Sitzfläche nach vorne und hinten verstellbar sein, wodurch eine der beiden Kreuzführungen eingespart werden kann; es ist dann nur noch eine Führung des Hydraulikzylinders 2 an dem Stuhl 1 erforderlich, die in senkrechter Richtung liegt, um eine Verstellung nach oben und unten zu ermöglichen.

Der Hydraulikzylinder ist in Fig. 2 schematisch dargestellt. In dem Hydraulikzylinder 2 ist ein beidseitig wirkender Kolben angeordnet, der aus den beiden Kolbenscheiben 5, 6 besteht, die durch die Zahnstange 7 starr miteinander verbunden sind. In gegenüberliegenden Bereichen der Zylinderwandung ist eine Schwenkachse 30 gelagert, auf die das Ritzel 8 aufgekeilt ist. Das Ritzel 8 kämmt mit der Zahnstange 7. Außerhalb des Zylinders 2 ist auf die Schwenkachse 30 der Schwenkhebel 3 aufgekeilt. Die durch die Kolbenscheiben 5, 6 abgeteilten Zylinderkammern 9, 10 sind durch einen Kanal 11 miteinander verbunden, in dem ein Drosselventil 12 angeordnet ist.

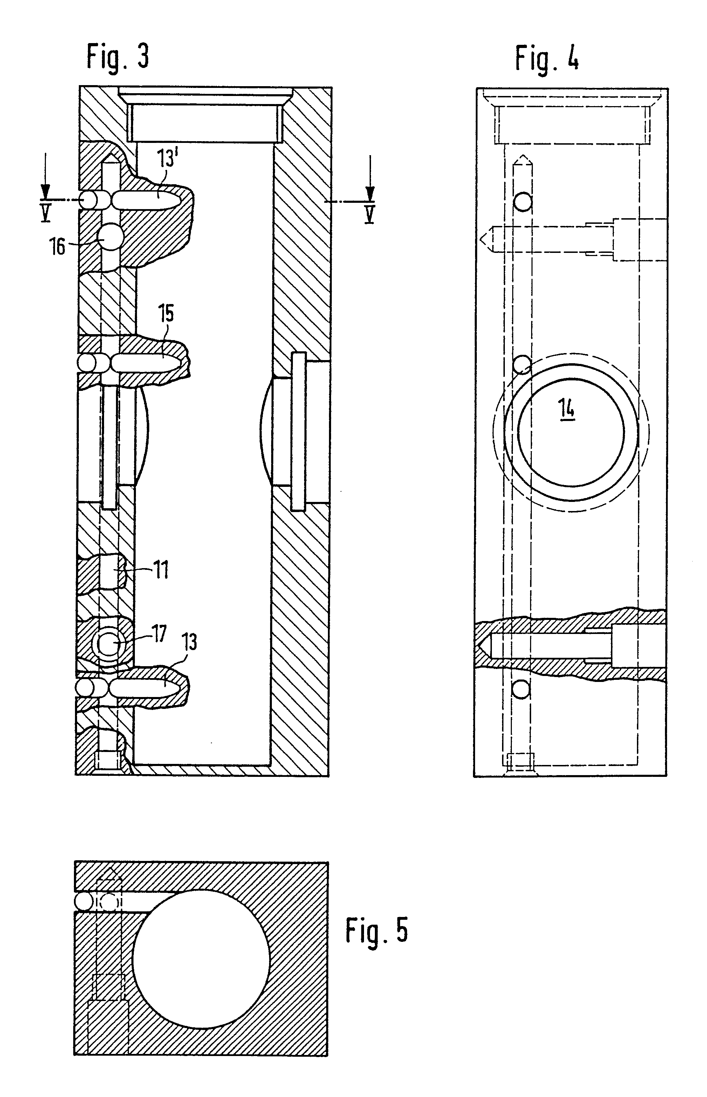

Bei dem aus den Fig. 3 bis 5 ersichtlichen Zylinder 2 ist der Drosselkanal 11 in der Zylinderwandung angeordnet. In den Endbereichen ist der Kanal mit Bohrungen 13, 13 versehen, die in die Zylinderkammern 9, 10 münden.

Der Zylinder weist in seinem mittleren Bereich eine Querbohrung 14 auf, in der die Schwenkwelle mit dem Ritzel gelagert ist, das mit der Zahnstange kämmt.

Weiterhin weist der Kanal 11 in seinem mittleren Bereich neben der Querbohrung 14 eine Querbohrung 15 auf, die in die Kolbenkammer zwischen den Kolbenscheiben 5, 6 mündet.

In seinen Endbereichen ist der Kanal von Gewindebohrungen 16, 17 durchsetzt, in die Drosselschrauben einschraubbar sind, durch die sich der jeweilige Drosselquerschnitt einstellen läßt.

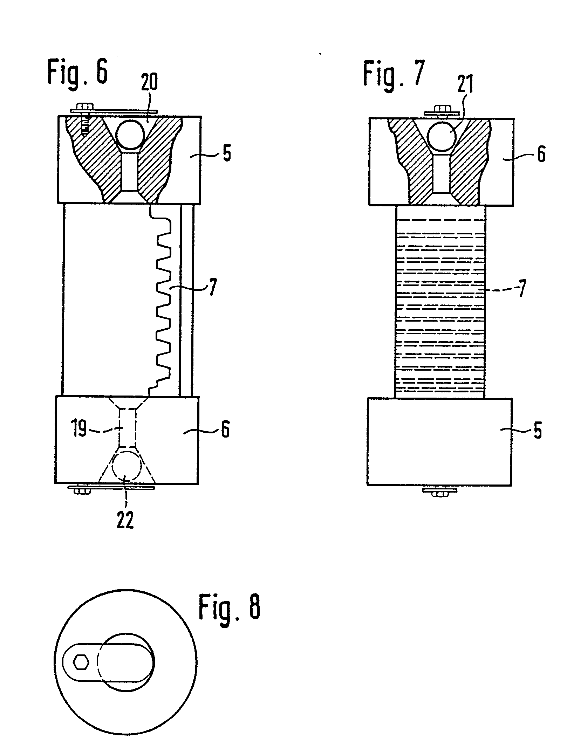

Die Ausgestaltung des Kolbens ist aus den Fig. 6 bis 8 näher ersichtlich. Der Kolben besteht aus den endseitigen Kolbenscheiben 5, 6, die durch die Zahnstange 7 miteinander verbunden sind. Die Kolbenscheiben 5, 6 sind mit abgesetzten Axialbohrungen 19, 20 versehen, in die Rückschlagventile bildende Stahlkugeln 21, 22 eingesetzt sind. Diese Stahlkugeln sind durch aufgeschraubte Sicherungsbleche in der dargestellten Weise gegen ein Herausfallen gesichert.

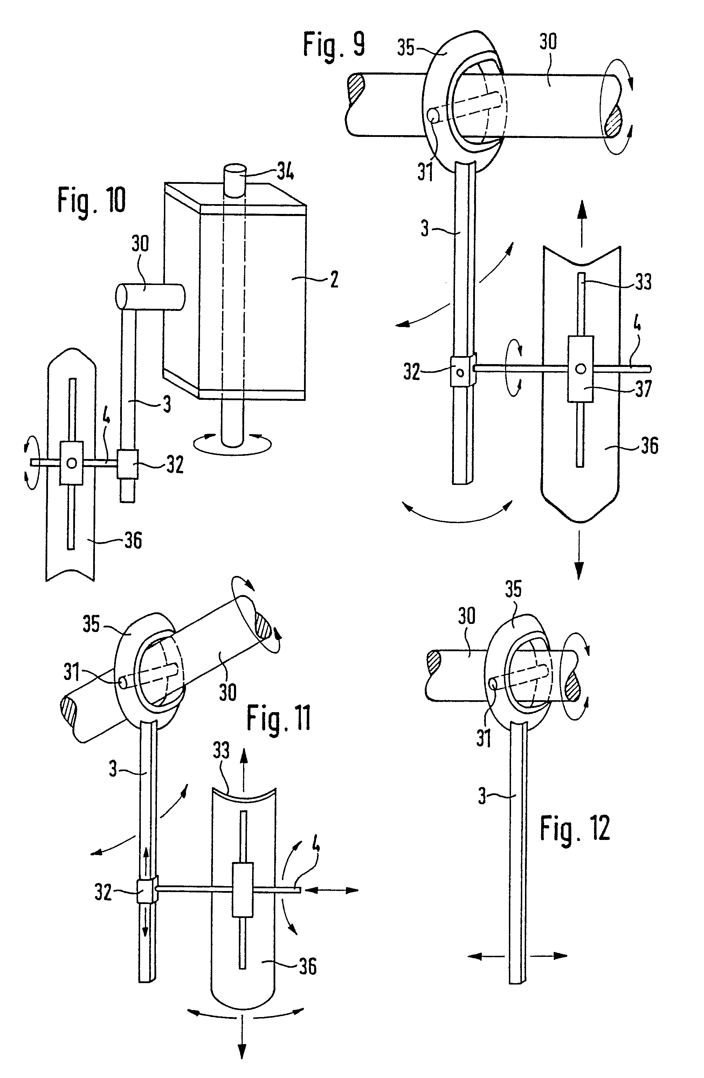

Fig. 9 zeigt in einer perspektivischen Darstellung die Schwenkachse 30, die beispielsweise auch in den Figuren 1 und 2 dargestellt ist. An der Schwenkachse 30 ist der Hebel 3 um eine Drehachse 31 schwenkbar gelagert. Die Drehachse 31 kann beispielsweise durch einen Bolzen gebildet werden, der die Schwenkachse 30 quer durchsetzt und auf zwei Seiten in Löcher des Auges 35 eingreift, wobei das Auge 35 mit dem Hebel 3 starr verbunden ist. Wie aus der Fig. 9 ersichtlich, steht die Drehachse 31 senkrecht zur Schwenkachse 30 und senkrecht zum Hebel 3. An dem Hebel ist das Verstellstück 32 verschieblich geführt. Dieses Verstellstück 32 dient zur Anpassung der Stellung der Beinaufnehmerschale 36. Je nach Körpergröße bzw. individuellen Wünschen kann das Verstellstück 32 zusammen mit der Beinaufnehmerschale 36 verschoben werden, bis die gewünschte Stellung erreicht ist. Dann wird das Verstellstück 32 mit Hilfe einer in der Zeichnung nicht dargestellten Klemmvorrichtung an dem Hebel 3 festgeklemmt. Mit dem Verstellstück 32 ist das Querstück 4 für die Beinaufnehmerschale 36 fest verbunden. Auf dem Querstück 4 ist das Zwischenstück 37 längsverschieblich und drehbar gelagert. Dieses Zwischenstück 37 kann nach Erreichen der gewünschten Winkelstellung und Längsstellung festgeklemmt werden. An dem Zwischenstück 37 ist die Verstellschiene 33 für die Beinaufnehmerschale 36 längsverschieblich gelagert. Nach Erreichen der gewünschten Stellung kann die Verstellschiene 33 und damit die Beinaufnehmerschale 36 festgeklemmt werden (in der Zeichnung nicht dargestellt). Die Bewegungsmöglichkeiten bzw. Verstellmöglichkeiten sind in der Fig. 9 mit Pfeilen angedeutet. In der Fig. 9 verläuft die Drehachse 31 senkrecht zur Schwenkachse 30 und senkrecht zum Hebel 3. Es ist jedoch auch möglich, daß die Drehachse nicht genau senkrecht, sondern geneigt ist.

Das Auge 35 des Hebels 3 ist in den Bereichen, in denen es bei einer Schwenkbewegung um die Drehachse 31 mit der Schwenkachse 30 in Berührung kommt, abgeschrägt, um Beschädigungen der Schwenkachse 30 zu vermeiden. Durch die Bemessung des Auges 35 bzw. der soeben beschriebenen Abschrägungen kann eine Winkelbegrenzung für die Drehbewegung um die Drehachse 31 vorherbestimmt werden.

Fig. 10 zeigt ein Ausführungsbeispiel einer zweiten Lösung für die Auslenkbarkeit des Hebels 3 in einer Richtung senkrecht zu seiner Bewegungsrichtung. An der Schwenkachse 30 ist der Hebel 3 starr befestigt. An dem Hebel 3 ist das Verstellstück 32 gleitbeweglich und feststellbar gelagert. Mit dem Gleitstück 32 ist das Querstück 4 für die Beinaufnehmerschale 36 fest verbunden. Der Zylinder 2 und dadurch auch die Schwenkachse 30 ist um die Drehachse 34 schwenkbar gelagert. Die Drehachse 34 verläuft senkrecht zur Schwenkachse 30 und im wesentlichen parallel zum Hebel 3. Genauer gesagt verläuft die Drehachse 34 dann parallel zum Hebel 3, wenn sich dieser Hebel 3 in seiner neutralen Mittelstellung befindet. Wenn der Hebel 3 um die Schwenkachse 30 im Verlaufe der Übung gedreht wird, nehmen die Drehachse 34 und der Hebel 3 einen Winkel zueinander ein, der dem Drehwinkel der Schwenkachse 30 entspricht. Die Anordnung kann auch so getroffen werden, daß die Drehachse 34 dann parallel zum Hebel 3 verläuft, wenn dieser Hebel 3 aus seiner neutra len Mittelstellung um einen bestimmten Winkel herausgeschwenkt worden ist. Weiterhin muß die Drehachse 34 nicht unbedingt genau senkrecht zur Schwenkachse 30 und auch nicht unbedingt parallel zum Hebel 3 verlaufen.

Fig. 11 zeigt eine weitere perspektivische Darstellung, die der Fig. 9 im wesentlichen entspricht und in der gleiche Teile mit gleichen Bezugszeichen versehen sind, so daß sich eine erneute Beschreibung erübrigt.

Fig. 12 zeigt eine Einzelheit aus der Fig. 11, wobei wiederum gleiche Teile mit gleichen Bezugszeichen versehen sind.

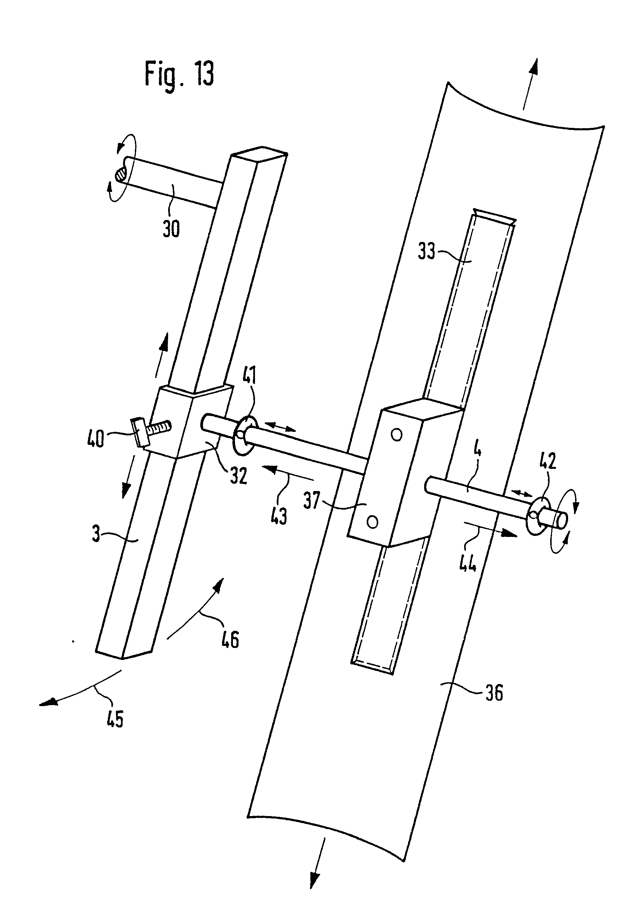

Bei der in Fig. 13 dargestellten Ausführungsform ist der Hebel 3 mit der Schwenkachse 30 fest verbunden. An dem Hebel 3 ist das Verstellstück 32 längsverschieblich geführt. Das Verstellstück 32 kann an dem Hebel 3 durch die Feststellschraube 40 festgeklemmt werden. Durch eine Drehung der Feststellschraube 40 wird ein Zwischenstück (in der Zeichnung nicht dargestellt) vorwärts bewegt und mit dem Hebel 3 zur Anlage gebracht. Vorzugsweise besitzt dieses Zwischenstück zwei senkrecht zueinander stehende Klemmflächen, die längs einer Kante des Hebels 3 auf zwei aneinandergrenzende Flächen des Hebels 3 durch die Kraft der Verstellschraube 40 aufgepreßt werden, wodurch die Festklemmung des Verstellstücks 32 auf dem Hebel 3 bewirkt wird.

Mit dem Verstellstück 32 ist das Querstück 4 für die Beinaufnehmerschale 36 fest verbunden. Auf dem Querstück 4 ist das Zwischenstück 37 längsverschieblich und drehbar gelagert. Im Gegensatz zur Ausführungsform nach der Fig. 9 wird das Zwischenstück 37 jedoch nicht auf dem Querstück 4 festgeklemmt, sondern kann sich auch während des Betriebs in Richtung des Querstücks 4 bewegen. Die Beinaufnehmerschale 36 ist also in einer Richtung 43, 44 senkrecht zur Bewegungsrichtung 45, 46 des Hebels 3 aus lenkbar. Die Bewegungsmöglichkeit des Zwischenstücks 37 ist durch zwei Anschläge 41 und 42 begrenzt. Die Anschläge 41, 42 können verschieblich und festklemmbar ausgestaltet sein. Auf der Beinaufnehmerschale 36 ist eine Verstellschiene 33 vorgesehen, die mit dem Zwischenstück 37 in Eingriff steht. Über die Verstellschiene 33 ist die Beinaufnehmerschale 36 mit dem Zwischenstück 37 längsverschieblich und festklemmbar verbunden.

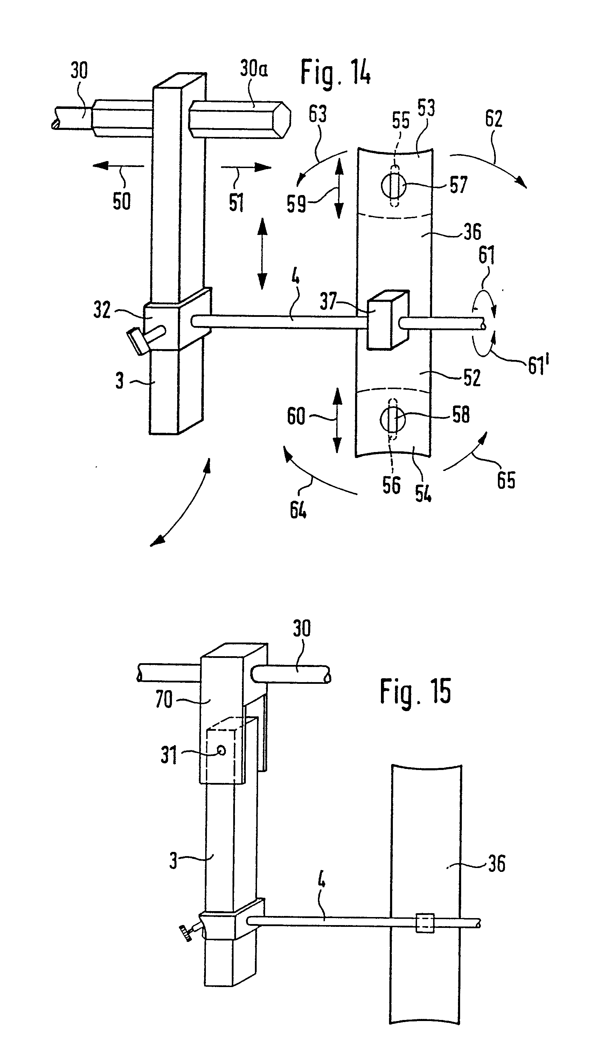

Bei der in Fig. 14 dargestellten Lösung ist die Schwenkachse 30 als Sechskantprofil 30a ausgestaltet. Der Hebel 3 ist also mit der Schwenkachse 30 drehfest und in Richtung der Schwenkachse längersverschieblich verbunden. Die Längsverschieblichkeit in Richtung der Schwenkachse ist durch die Pfeile 50, 51 angedeutet. Die übrigen Bezugsziffern der Fig. 14 entsprechen denjenigen der anderen Figuren und brauchen daher nicht mehr im einzelnen erläutert zu werden. Das Zwischenstück 37 wird auf dem Querstück 4 in die richtige Stellung geschoben und dann festgeklemmt. Die Beinaufnehmerschale 36 besteht aus einem Mittelstück 52 und zwei Endstücken 53 und 54. In dem Mittelstück 52 befinden sich zwei Langlöcher 55 und 56. In den Endstücken 53 und 54 sind Löcher 57 und 58 vorgesehen. Die Endstücke 53 und 54 können also in Richtung der Doppelpfeile 59 und 60 verschoben und einjustiert werden.

Das Zwischenstück 37 ist auf dem Querstück 4 drehbar gelagert; siehe Pfeile 60, 61. Die Beinaufnehmerschale 36 ist an dem Zwischenstück 37 ebenfalls drehbar gelagert; siehe Pfeile 62 - 65.

Bei der in Fig. 15 dargestellten Ausführungsform ist ein Zwischenstück 70 mit der Schwenkachse 30 fest verbunden. Das Zwischenstück 70 ist in seinem unteren Bereich U-förmig ausgebildet und trägt dort die Drehachse 31, an der der Hebel 3 schwenkbar gelagert ist. Die Drehachse 31 verläuft senkrecht zur Schwenkachse 30. Die Drehachse 31 verläuft weiterhin im Abstand zur Schwenkachse 30.

Bei sämtlichen Ausführungsformen kann die Drehachse 31 geschwindigkeitsabhängig gedämpft und/oder geschwindigkeitsunabhängig belastet sein. Diese Dämpfung und/oder Belastung kann symmetrisch oder unsymmetrisch sein. Durch eine unsymmetrische Vorbelastung kann eine Seite des beanspruchten Gelenkes entlastet werden.

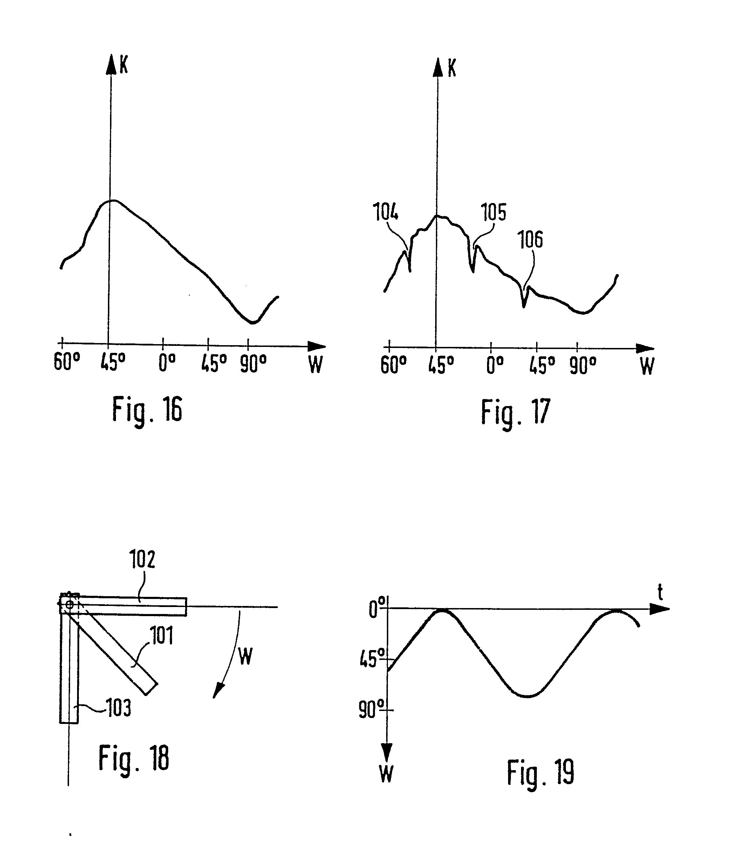

In der Fig. 16 ist ein Diagramm gezeigt, bei dem auf der horizontalen Achse der Winkel w und auf der vertikalen Achse die Kraft K aufgetragen ist. Die Kraft K kann dabei aus dem in dem Zylinder herrschenden Druck und der (bekannten) Kolbenfläche berechnet werden. Der Druck im dem Zylinder wird durch eine den Druck der hydraulischen Flüssigkeit messende Einrichtung gemessen. Diese Einrichtung kann dem Patienten oder dem Arzt das jeweilige Reaktionsmoment anzeigen. Zustätzlich kann eine Einrichtung vorgesehen sein, die den gemessenen Druck anzeigt und/oder aus diesem die Arbeit und/oder die Leistung ermittelt und diese anzeigt und/oder registriert. Die von dem Patienten geleistete Arbeit läßt sich aus dem jeweiligen Widerstand und den Schwenkwinkeln errechnen, wobei zur Ermittlung der Leistung noch die Winkelgeschwindigkeit zu messen ist. Der Winkel und die Winkelgeschwindigkeit werden durch einen Winkelgeber gemessen.

Auch eine Datenspeicherung zur Statistik und Programmgestaltung ist möglich. Die Drehbewegung kann mittels Drehgeber registriert und auf einer Digital-oder Analoganzeige sichtbar gemacht werden, die von jeder beliebigen Position auf Ausgangs-oder Endstellung gestellt werden kann. Der Öldruck, also der Druck in der Zylinderkammer, kann von Drucksensoren erfaßt und entweder digital oder analog sichtbar gemacht werden. Die erfaßten Meßwerte können direkt einer Datenverarbeitungsanlage zugeführt, gespeichert, ausgewertet und ausgedruckt werden. Mit Hilfe eines speziell erarbeiteten Programms lassen sich die medizinischen Daten des Patienten eingeben, beispielsweise das Gewicht des aus der Streckung völlig entspannt zurückfallenden Unterschenkels.

Die Fig. 18 zeigt den Hebel in drei verschiedenen Stellungen. In der mit 102 bezeichneten Stellung ist das Knie gestreckt; der Beugungswinkel beträgt 0°. In der Stellung 101 ist das Knie halb angewinkelt; der Beugungswinkel w beträgt 45°. In der mit 103 bezeichneten Stellung ist das Knie voll angewinkelt; der Beugungswinkel w beträgt 90°.

In der Fig. 16 ist die über den Druck in dem Zylinder ermittelte Kraft K abhängig von dem Winkel w dargestellt. Die in der Fig. 16 und auch in der Fig. 17 dargestellten Kurven können direkt von einem Oszillographen erzeugt und auf dessen Bildschirm gesehen werden. Sie können auch über einen Drucker oder Plotter auf Papier oder ein ähnliches Druckmedium geschrieben werden.

Wie aus der Fig. 16 ersichtlich, beginnt die Meßschreibung bei einer Winkelstellung von 60° in Richtung auf abnehmende Winkel. Der Winkel nimmt bis zu 0° ab (siehe Bezugsziffer 102 in Fig.18) und steigt dann wieder bis 90° an (siehe Bezugsziffer 103 in Fig. 18). Anschließend nimmt der Winkel wieder ab, und ein neuer Zyklus beginnt.

Die Meßschreibung muß nicht bei einer Winkelstellung von 60° beginnen, sondern kann auf einen beliebigen Winkel voreingestellt werden. Der Start der Messung erfolgt durch den Null-Impuls des Drehgebers, der auf einen beliebigen Winkel voreingestellt werden kann.

Die Abtastfrequenz beträgt vorzugsweise 1000 Hz. D.h., es werden jede Sekunde 1000 Meßpunkte (Druck/Winkel) in digitaler Form in einer RAM-Floppy abgelegt. Durch Verändern des Ausleseabstandes (Step) kann die Kurve nach Belieben verkleinert werden.

Die gemessenen Werte können abgespeichert werden, beispielsweise auf einer Floppy-Disk. Die abgespeicherten Werte können dann von einem Rechner, beispielsweise einem Personal-Computer, abgerufen und weiterverarbeitet werden.

Die Fig. 16 zeigt einen Druckverlauf für ein Gelenk, dessen Gelenkstruktur keine Auffälligkeiten zeigt. In der Fig. 17 ist ein Kraftverlauf gezeigt, der bei der Diagnose eines Gelenkes ermittelt wurde, dessen Gelenkstruktur Abweichungen von der Normalstruktur aufweist. Diese Abweichungen können anhand der "Druckeinbrüche" 104, 105 und 106 ermittelt werden. Die Anzahl, Lage und Ausgestaltung dieser Druckeinbrüche ermöglichen es dem Arzt, die Art und Ausgestaltung der Abweichung in der Gelenkstruktur von der normalen Gelenkstruktur zu diagnostizieren.

Es ist auch möglich, bei einer Person zunächst einen Kraftverlauf der in den Fig. 16 oder 17 dargestellten Art aufzunehmen und dann aufzubewahren. Darauf kann dann bei späteren Diagnosen als Referenzkurve zurückgegriffen werden. So ist es dann möglich, die Auswirkungen einer zwischenzeitlichen Therapie oder auch eines zwischenzeitlichen Trainings festzustellen. Dieses Training kann ein Rehabilitationstraining sein, es kann aber auch das Leistungstraining eines Sportlers sein.

Die Fig. 19 zeigt einen typischen Verlauf der Winkelstellung w in Abhängigkeit von der Zeit t. Wie aus der in der Fig. 19 dargestellten Kurve unmittelbar ersichtlich, beginnt die Übung bei der Winkelstellung von 60° und geht zunächst in Richtung auf abnehmende Winkel. Wenn die Winkelstellung von 0° erreicht ist, steigt anschließend der Winkel wieder an, bis er 90° erreicht. Anschließend nimmt der Winkel wieder ab, und ein neuer Zyklus beginnt.

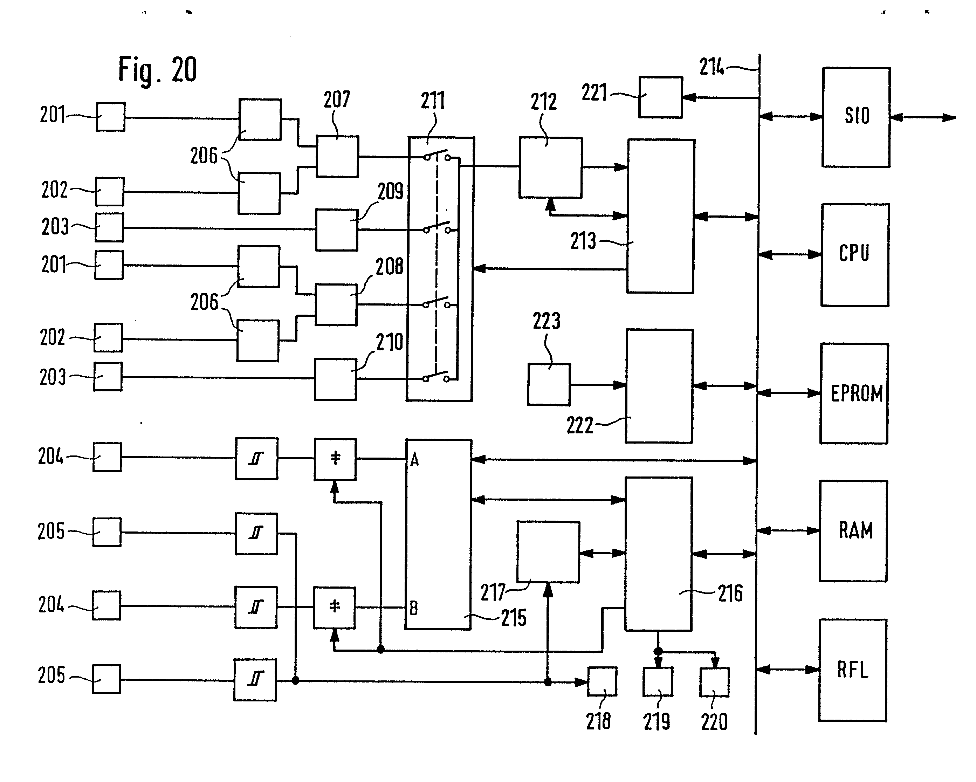

Die Fig. 20 zeigt schematisch den prinzipiellen Aufbau eines Auswertegerätes. An dem Stuhl 1 sind sowohl auf der linken Seite als auch auf der rechten Seite jeweils ein Hydraulikzylinder 2 für das linke und für das rechte Bein befestigt. Jeder Zylinder besitzt auf jeder Seite einen Druckgeber 201 für die Beugung und einen Druckgeber 202 für die Streckung. In jedem Zylinder 2 ist weiterhin ein Temperaturfühler 203 vorgesehen.

Jeder der beiden Zylinder 2 weist weiterhin einen Winkelgeber 204 auf, der ein Maß für die Winkelstellung des Schwenkhebels 3 relativ zum Zylinder 2 liefert. Weiterhin kann über die Referenzgeber 205, die in den Winkelgebern integriert sein können, eine Referenzmarke für die Winkelstellung eingestellt werden. Bei der eingestellten Referenzmarke beginnt die Messung.

Die von den Druckgebern kommenden Stromsignale werden durch I-U-Wandler 206 in Spannungssignale umgewandelt. Das so umgewandelte Spannungssignal des linken Druckgebers 201 für die Beugung und des linken Druckgebers 202 für die Streckung werden einem Summierverstärker 207 zugeführt. Gleichermaßen werden die entsprechenden Drucksignale für den auf der rechten Seite des Stuhles 1 angeordneten Zylinder 2 einem weitern Summierverstärker 208 zugeführt.

Die Temperatursignale werden ebenfalls durch die I-U-Wandler 209, 210 von einem Stromsignal in ein Spannungssignal umgewandelt.

Die umgewandelten und summierten Drucksignale und die umgewandelten Temperatursignale werden einem Analog-Multiplexer 211 zugeführt, der diese Signale an einen 10 Bit Analog-Digital-Wandler 212 weiterleitet. Die derart digitalisierten Signale werden dann an eine Ein-/Ausgabe-Einheit 213 übergeben, die mit dem Systembus 214 in Verbindung steht.

Die Winkelsignale 204 werden einem Zählerbaustein 215 zugeleitet, der sowohl mit dem Systembus 214 als auch mit der Ein-/Ausgabe-Einheit 216 in Verbindung steht. Die Referenzmarken 205 sind über eine Startlogik 217 mit der Ein-Ausgabe-Einheit 216 verbunden. Weiterhin werden die Referenzmarken-Signale einer Startimpuls-Anzeige-Einheit 218 zugeführt, die aus einer Leuchtdiode bestehen kann. Die Ein-/Ausgabe-Einheit 216 ist mit zwei Leuchtdioden 219, 220 verbunden, die anzeigen, auf welcher Seite (links/rechts) gerade gemessen wird.

Das Auswertegerät wird durch die Zentraleinheit (Steuerrechner) CPU gesteuert. Ein Festwertspeicher EPROM dient zur Speicherung der Betriebssoftware, ein Schreib-Lese-Speicher RAM dient als Hilfsspeicher. Die Meßwerte können in den Meßwertespeicher RFL abgelegt werden, der aus einem RAM Floppy Speicher bestehen kann. Über die serielle Schnittstelle SIO wird das Auswertegerät mit einem Auswerterechner, beispielsweise einem Personal-Computer, verbunden. Das Auswertegerät wird durch den Personal-Computer gesteuert. In diesem Auswerterechner können die in dem Meßwertespeicher RFL abgelegten Daten weiterverarbeitet und evtl. auf einer Floppy Disk abgelegt werden.

Eine weitere Leuchtdiode 221 dient zur Anzeige eines Bereitschaftssignals.

Mit dem Systembus 214 ist ein Zeitgeber (Timer) 222 für die Baudrate und den Meßtakt verbunden. Der Zeitgeber empfängt Takte von einem Taktgeber 223.

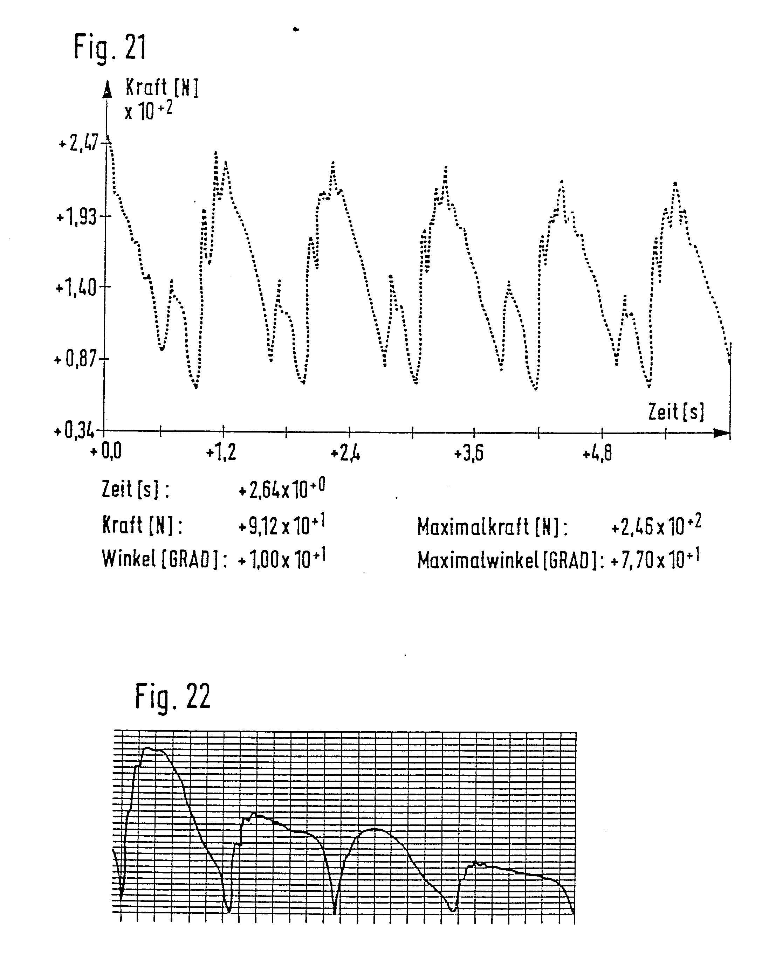

Die Fig. 21 zeigt einer Original-Meßkurve, die mit dem soeben beschriebenen Diagnosegerät und dem zugehörigen Auswertegerät erstellt worden ist. Die Meßkurve ist aus einzelnen, dicht aufeinander folgenden Meßpunkten zusammengesetzt. Durch die hohe Abtastfrequenz ist diese Meßkurve sehr genau, weshalb auch kleinste Druckeinbrüche erkannt werden können.

Die Fig. 22 zeigt eine Meßkurve, die mit einem vorbekannten Gerät hergestellt worden ist. Die mit diesem Gerät erzielbare Genauigkeit reicht für eine zuverlässige Diagnose nicht aus.

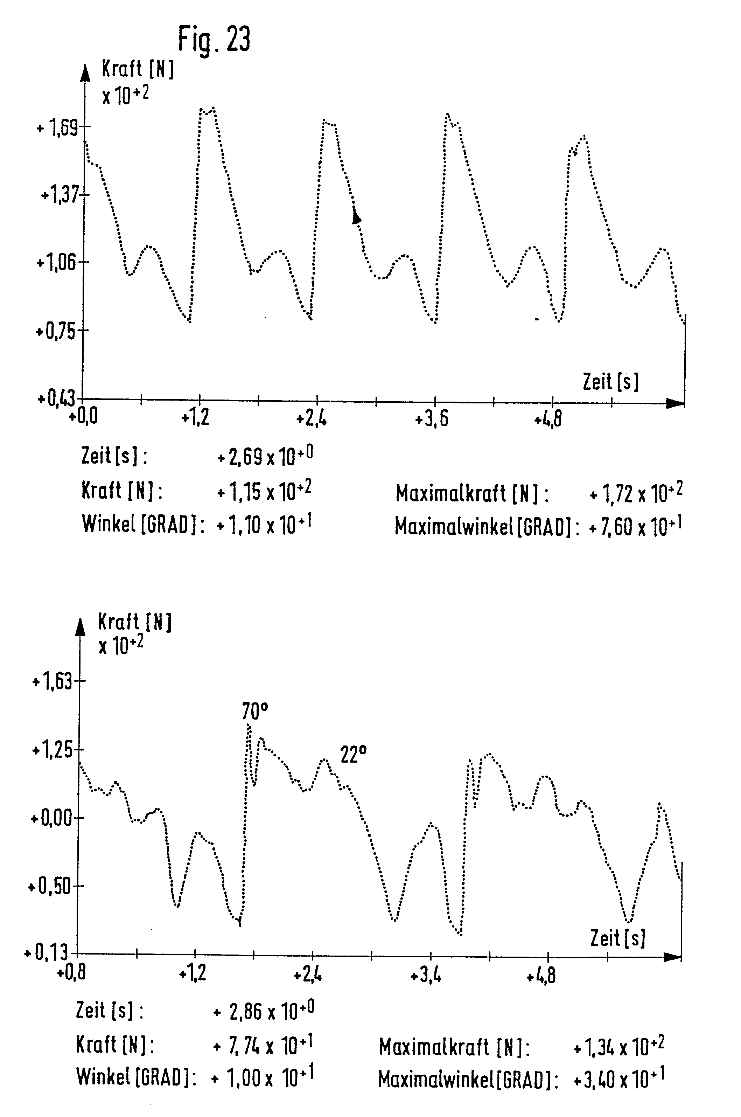

Die Fig. 23 zeigt zwei weitere mit dem erfindungsgemäßen Diagnosegerät hergestellte Original-Meßkurven. Die obere Kurve zeigt den Durckverlauf bei einer Bewegung des rechten Körpergelenks der Testperson, die untere Kurve die Bewegung des linken Körpergelenks, Lage und Ausmaß der verschiedenen Druckeinbrüche können sehr genau festgestellt werden, was eine zuverlässige Diagnose ermöglicht. Mit der sogenannten "MAUS" des an das Auswertesystem angeschlossenen Personal-Computers kann der in den beiden Meßkurven sichtbare Pfeil exakt positioniert werden. Dadurch können für jeden Punkt der Meßkurve die Koordinaten zuverlässig bestimmt und in digitaler Form ausgegeben und angezeigt werden. Hierdurch wird eine exakte Diagnose erleichtert und in manchen Fällen überhaupt erst ermöglicht.

Durch das erfindungsgemäße medizinische Diagnosegerät kann eine durch eine Testperson erbrachte Folge von Bewegungen in kleinen Schritten aufgezeichnet werden. Dies ermöglicht der auswertenden Person eine zuverlässige Diagnose. Es können mit ständig unterschiedlicher Winkelgeschwindigkeit, die nur durch den voreingestellten Drosselspalt und die am Schwenkhebel anliegende Kraft bestimmt ist, dynamisch über einen wählbaren relativ kurzen bis etwa gut 1 Minute und mehr betragenden Zeitraum alle Bewegungen der Testperson, z.B. Flexionen und Extensionen am Kniegelenk, exakt aufgezeichnet werden. Eine darstellende Graphik vermittelt dem Therapeuten die Gesamtübersicht, aus der er jede für ihn interessante Stelle genauer betrachten, analysieren und dokumentieren kann - er kann über alle Daten ohne Einschränkung verfügen, weil sie in digitaler Form fest gespeichert vorliegen und durch entsprechende Software verarbeitet werden können. Durch eine genaue Sitzpositionierung kann eine gedachte Gelenksmittendrehachse mit der Antriebsdrehachse bestmöglich fluchten. Auch soll die Testperson bequem sitzen können und weder am zu prüfenden Gelenk noch an anderen Körperteilen unnötig belastet oder gar geschädigt werden. Hierzu dient insbesondere die "kardanische Aufhängung", also das Merkmal, daß der Hebel in einer Richtung senkrecht zu seiner Bewegungsrichtung auslenkbar ist.

## Ansprüche

1. Medizinisches Diagnosegerät mit einem an einer Schwenkachse (30) befestigten Hebel (3), der zur Erzeugung eines Reaktionsmoments mit dem Kolben (5, 6) eines Hydraulikzylinders (2) so verbunden ist, daß eine alternierende Drehbewegung des Hebels (3) in eine translatorische hin-und hergehende Bewegung des Kolbens (5, 6) umgewandet wird, mit einer Meßeinrichtung zum Messen des in dem Hydraulikzylinder herrschenden Drucks und mit einer Anzeigeeinrichtung zum Anzeigen des gemessenen Drucks, dadurch gekennzeichnet, daß die Schwenkachse (30) den Zylinder (2) quer durchsetzt und daß die Schwenkachse (30) im Innern des Zylinders (2) mit dem Kolben (5, 6) durch ein Getriebe (7, 8) verbunden ist.

2. Diagnosegerät nach Anspruch 1, gekennzeichnet durch einen doppelt wirkenden Kolben (5, 6) und einen eine Drosseleinrichtung (12) aufweisenden Kanal (10), der die durch den Kolben (5, 6) getrennten und mit Flüssigkeit gefüllten Zylinderkammern (9, 10) miteinander verbindet.

3. Diagnosegerät nach Anspruch 1 oder 2, gekennzeichnet durch eine Winkelmeßvorrichtung zur Messung des Drehwinkels der Schwenkachse (30).

4. Diagnosegerät nach einem der vorhergehenden Ansprüche, dadurch gekennzeichnet, daß in den Endbereichen des Kanals je ein einstellbares Drosselventil (16, 17) angeordnet ist, daß der Kolben aus zwei starr miteinander verbundenen Kolbenscheiben (5, 6) besteht, die zwischen sich eine Kolbenkammer begrenzen, daß in jeder Kolbenscheibe ein einen Durchfluß in die angrenzende Zylinderkammer (9, 10) gestattendes Rückschlagventil (21, 22) angeordnet ist und daß der Kanal zwischen den Drosselventilen mit einer durch die Zylinderwandung hindurch in die Kolbenkammer mündenden Leitung versehen ist.

5. Diagnosegerät nach einem der vorhergehenden Ansprüche, dadurch gekennzeichnet, daß der Hebel in einer Richtung senkrecht zu seiner Bewegungsrichtung auslenkbar ist.

6. Diagnosegerät nach einem der vorhergehenden Ansprüche, gekennzeichnet durch eine Verarbeitungseinrichtung zur Verarbeitung der Druckmeßwerte und/oder der Winkelmeßwerte.

7. Diagnosegerät nach einem der vorhergehenden Ansprüche, dadurch gekennzeichnet, daß die Anzeigeeinrichtung aus einem Oszillographen und/oder einem Meßschreiber besteht.

8. Diagnosegerät nach einem der vorhergehenden Ansprüche, dadurch gekennzeichnet, daß der Zylinder mit der Schwenkachse eines zweiten gleichartigen, an dem Diagnosegerät befestigten Zylinders verbunden ist, wobei die beiden Achsen miteinander fluchten oder im Winkel zueinander angeordnet sind.

9. Diagnosegerät nach einem der vorhergehenden Ansprüche, gekennzeichnet durch einen Analog-Digital-Wandler, einen Meßwertspeicher, einen oder mehrere Druckmeßgeber, einen Analog-Muitiplexer, einen oder mehrere Winkelgeber, einen Zählerbaustein, einen Steuerrechner, einen Festspeicher, einen Schreib-Lesespeicher und/oder eine serielle Schnittstelle.

10. Diagnosegerät nach einem der vorhergehenden Ansprüche, dadurch gekennzeichnet, daß die Meßeinrichtung jeweils einen Druckgeber auf jeder der beiden Seiten des Kolbens umfaßt.

Fig. 1

Fig. 2

Fig. 3

13'

16

15

11

17  13

Fig. 4

14

Fig. 5

Fig. 6

20

5

7

19

6

22

Fig. 7

21

6

7

5

Fig. 8

0 275 956

Fig. 9

Fig. 10

Fig. 11

Fig. 12

## Fig. 13

Fig. 14

Fig. 15

Fig. 16

Fig. 17

Fig. 18

Fig. 19

Fig. 20

0 275 956

## Fig. 21

Zeit [s] :          $+2{,}64 \times 10^{+0}$

Kraft [N] :         $+9{,}12 \times 10^{+1}$          Maximalkraft [N] :          $+2{,}46 \times 10^{+2}$

Winkel [GRAD] : $+1{,}00 \times 10^{+1}$          Maximalwinkel [GRAD] : $+7{,}70 \times 10^{+1}$

## Fig. 22

## Fig. 23

Zeit [s] :  $+2{,}69 \times 10^{+0}$

Kraft [N] :  $+1{,}15 \times 10^{+2}$    Maximalkraft [N] :  $+1{,}72 \times 10^{+2}$

Winkel [GRAD] :  $+1{,}10 \times 10^{+1}$    Maximalwinkel [GRAD] :  $+7{,}60 \times 10^{+1}$

Zeit [s] :  $+2{,}86 \times 10^{+0}$

Kraft [N] :  $+7{,}74 \times 10^{+1}$    Maximalkraft [N] :  $+1{,}34 \times 10^{+2}$

Winkel [GRAD] :  $+1{,}00 \times 10^{+1}$    Maximalwinkel [GRAD] :  $+3{,}40 \times 10^{+1}$